(19) Europäisches Patentamt
European Patent Office
Office européen des brevets

(11) Numéro de publication: **0 133 176**
**B1**

(12) **FASCICULE DE BREVET EUROPÉEN**

(45) Date de publication du fascicule du brevet: **03.09.86**

(51) Int. Cl.⁴: **C 07 D 307/80**, A 61 K 31/34

(21) Numéro de dépôt: **84870101.7**

(22) Date de dépôt: **12.07.84**

(54) **Composition pharmaceutique ou vétérinaire destinée à combattre des troubles ischémiques cardiaques.**

(30) Priorité: **02.08.83 FR 8312739**

(43) Date de publication de la demande: **13.02.85 Bulletin 85/7**

(45) Mention de la délivrance du brevet: **03.09.86 Bulletin 86/36**

(84) Etats contractants désignés: **CH DE FR GB IT LI LU NL SE**

(56) Documents cité:
**FR-M-2 280**

**JOURNAL OF CHROMATOGRAPHY, vol. 273, no. 2, 8 avril 1983, pages 379-392, Elsevier Scientific Publishing Co., Amsterdam, NL; T.A. PLOMP et al.: "Simultaneous determination of amiodarone and its major metabolite desethylamiodarone in plasma, urine and tissues by high-performance liquid chromatography"**
**CHEMICAL ABSTRACTS, vol. 98, no. 11, 14 mars 1983, page 6, no. 83156c, Columbus, Ohio, US; R.J. FLANAGAN et al.: "Identification and measurement of desethylamiodarone in blood plasma specimens from amiodarone-treated patients"**

(73) Titulaire: **SANOFI, société anonyme, 40, Avenue George V, F-75008 Paris (FR)**

(72) Inventeur: **Descamps, Marcel, Rue du Bois du Bosquet 30, B-1331 Rosieres (BE)**
Inventeur: **Berger, Yves, Avenue des Nerviens 16, B-1810 Wemmel (BE)**

(74) Mandataire: **Cauchie, Daniel, c/o S.A. LABAZ-SANOFI N.V. Avenue De Béjar, 1, B-1120 Bruxelles (BE)**

**EP 0 133 176 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention se rapporte, d'une manière générale, à une composition pharmaceutique ou vétérinaire destinée notamment à combattre des troubles ischémiques cardiaques.

En particulier, l'invention concerne une composition pharmaceutique ou vétérinaire contenant, comme principe actif essentiel, au moins un dérivé de benzofuranne de formule générale:

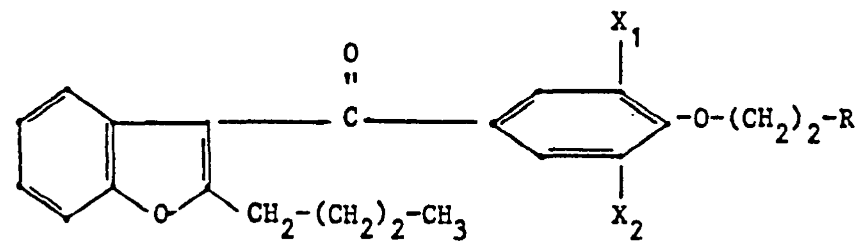

I

ou un de ses sels d'addition non toxique par exemple le chlorhydrate ou l'oxalate, dans laquelle R représente un radical amino, monoéthylamino ou diéthylamino et $X_1$ et $X_2$, qui sont identiques ou différents, représentent hydrogène ou iode à condition que lorsque R représente un radical diéthylamino, $X_1$ et $X_2$ sont différents. Ces dérivés de bensofuranne ainsi que leurs sels d'addition non toxiques sont désignés ci-après sous le terme général: composés selon l'invention.

On a trouvé que les composés selon l'invention sont doués d'intéressantes propriétés pharmacologiques dans le domaine cardiovasculaire. En particulier, les composés en question se sont montrés capables de diminuer la fréquence cardiaque et la pression artérielle ainsi que de corriger des arythmies cardiaques. En outre, les composés selon l'invention ont révélé de très intéressantes propriétés inhibitrices de la translocation calcique au niveau membranaire.

Les composés selon l'invention ainsi que les compositions pharmaceutiques ou vétérinaires les contenant seront, par conséquent, utiles en vue de combattre des troubles ischémiques cardiaques et, à cette fin, seront particulièrement indiqués dans le traitement d'arythmies cardiaques, de l'angor vasospastique, de l'angine d'effort ou de l'angine de poitrine double. Les composés de formule I ci-dessus sont connus. Ceux-ci ont été cités par FLANAGAN et coll. dans J. Pharm. Pharmacol. 1982, 34 pp. 638-643. Toutefois, aucune propriété pharmacologique ou application thérapeutique n'y a été révélée les concernant.

En outre, on a décrit dans le B.S.M. français No. 2.280 M, des dérivés de dialkylaminoalkoxy benzoyl-3 benzofuranne utiles comme agents antiangineux. Parmi ces composés figurent notamment le n-butyl-2[(diéthylamino-2 éthoxy)-4 diiodo-3,5 benzoyl]-3 benzofuranne ou amiodarone ainsi que le n-butyl-2 [(diéthylamino-2 éthoxy)-4 benzoyl]-3 benzofuranne, ci-après dénommé Composé A, ainsi que leurs sels d'addition non toxiques.

On a maintenant trouvé, de manière surprenante qu'en effectuant au moins une modification au niveau des deux composés antérieurs ci-dessus, c'est-à-dire en éliminant au moins un atome d'iode et/ou un radical éthyle au niveau du groupement diéthylamino, les composés ainsi formés présentent des propriétés inhibitrices de la translocation calcique supérieures à celles des deux composés antérieurs en question.

Cette découverte est d'autant plus remarquable qu'aucune allusion n'est faite, dans le B.S.M. No. 2.280 M, à d'éventuelles propriétés inhibitrices calciques en ce qui concerne les composés y décrits.

Les propriétés inhibitrices calciques des composés selon l'invention ont été mises en évidence au moyen du test décrit par POLSTER et coll. dans Biochemical Pharmacology vol. 30, No. 8 pp. 897-901 (1981) c'est-à-dire par mesure de l'action antagoniste des composés selon l'invention vis-à-vis de la réponse contractile à la dépolarisation provoquée par le potassium sur l'aorte isolée de rat.

Il est bien établi que la dépolarisation de la membrane d'un muscle lisse par le potassium rend cette dernière perméable au calcium extracellulaire et provoque la contraction musculaire. Dès lors, la mesure de l'inhibition de la réponse contractile à la dépolarisation par le potassium ou la mesure d'un relâchement de la contraction tonique à la dépolarisation potassique peut constituer une évaluation de la puissance d'un composé en tant qu'inhibiteur de la perméabilité membranaire aux ions $Ca^{++}$.

La technique utilisée est la suivante:

Sur des rats mâles Wistar pesant environ 300 g, on prélève l'aorte et on la coupe en bandelettes d'environ 40 mm de longueur et 3 mm de largeur. On place ces fragments dans une cuve à organe isolé de 25 ml contenant une solution de Krebs-bicarbonate modifiée (NaCl 112 mM; KCl 5 mM; $NaHCO_3$ 25 mM; $KH_2PO_4$ 1 mM; $MgSO_4$ 1,2 mM; $CaCl_2$ 2,5 mM; glucose 11,5 mM; eau distillée jusqu'à 1000 ml), parcourue par un courant de carbogène et maintenue à 37° C. On relie la préparation à un microcapteur de force et on enregistre la réponse contractile après amplification sur un enregistreur.

On applique une tension de 2 g à la préparation. On maintient la préparation durant 60 minutes dans la solution de Krebs-bicarbonate modifiée puis on provoque des contractions en remplaçant la solution Krebs-bicarbonate par une solution de Krebs-potassique (NaCl 17 mM; KCl 400 mM; $NaHCO_3$ 25 mM; $KH_2PO_4$ 1 mM; $MgSO_4$ 1,2 mM; $CaCl_2$ 2,5 mM; glucose 11,5 mM; eau distillée jusqu'à 1000 ml).

Lorsque la réponse contractile de la préparation est devenue reproductible on introduit, dans le bain, une quantité égale à $10^{-5}$ mole d'un composé selon l'invention.

Soixante minutes plus tard, un nouveau spasme est provoqué par la dépolarisation potassique.

Les résultats obtenus sur chaque aorte éprouvée sont alors exprimés en % de l'effet contracturant maximal avant l'incubation avec le composé à tester. A titre d'exemples, les résultats suivants ont été obtenus comparativement à l'amiodarone et au Composé A:

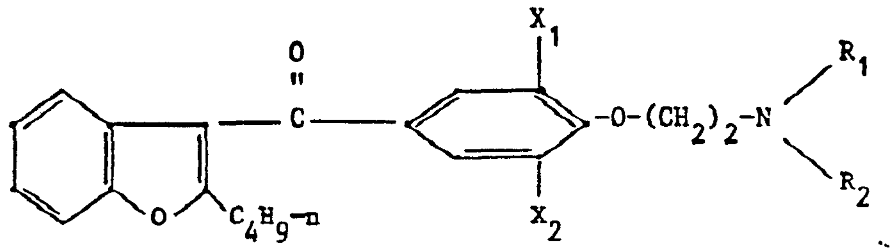

| Composé | $X_1$ | $X_2$ | $R_1$ | $R_2$ | % de l'effet contracturant maximal |
|---|---|---|---|---|---|
| 1 | I | I | H | $C_2H_5$ | 65,6 |
| 2 | I | I | H | H | 73,3 |
| 3 | I | H | H | H | 68,7 |
| 4 | I | H | H | $C_2H_5$ | 50,8 |
| 5 | H | H | H | $C_2H_5$ | 8,7* |
| | | | | | 13,6** |
| 6 | I | H | $C_2H_5$ | $C_2H_5$ | 67,6 |

Amiodarone 89,3
Composé A 20,4
*: resultat obtenu à partir du chlorhydrate
**: resultat obtenu à partir de l'oxalate acide

Ces résultats montrent que le passage de l'amiodarone aux Composés 1 à 6 par suppression d'atomes d'iode, et/ou de radicaux éthyle forment des dérivés de benzofuranne ayant des propriétés inhibitrices calciques supérieures à celles de l'amiodarone.

De même, la suppression d'un radical éthyle au niveau du Composé A donne naissance au Composé 5 présentant des propriétés imhibitrices calciques supérieures à celles du Composé A en question.

Des tests pharmacologiques complémentaires effectués chez le chien à la dose de 10 mg/kg par voie intraveineuse, ont montré que les Composés 1, 2, 4 et 5 sous forme de base ou de sel d'addition non toxique provoquent respectivement une dimimution de 24%, 25%, 25% et 17% de la fréquence cardiaque ainsi qu'une chute brève et modérée de la pression artérielle.

En outre, les Composés 4 et 5 se sont revélés capables, dans ces conditions, de diminuer la consommation en oxygène du myocarde.

Des études complémentaires ont, en outre, montré les propriétés antiarythmiques des composés selon l'invention vis-à-vis de diverses arythmies expérimentales, notamment vis-à-vis de l'arythmie provoquée, chez le rat anesthésié, par la ligature de l'artère coronaire gauche.

A cet effet, on a anesthésié à l'uréthane des lots de 4 à 6 rats mâles et on a posé un fil de ligature sous l'artère coronaire gauche à 1 mm en dessous de l'oreillette gauche. On a injecté le composé à étudier en 2 min. par une veine fémorale, 5 min. après la pose du fil et on a serré la ligature 5 min. après l'injection en question.

En fin d'essai, on a injecté, par voie veineuse, une solution de Bleu evans (0,2%-1 ml/100 g de rat) pour déterminer au niveau du ventricule gauche, l'importance de la zone non irriguée provoquée par la ligature.

On a ainsi constaté que les Composés 1 et 5 protègent, en administration aiguë, contre l'arythmie et la mortalité provoquées par une ligature coronaire chez le rat.

Déjà légèrement actifs à 5 mg/kg, les Composés 1 et 5 administrés par voie intraveineuse 5 minutes avant le serrage de la ligature protègent totalement 50% des animaux à 10 mg/kg et assurent une protection de 100% à 15 mg/kg. Des essais comparatifs effectués dans les mêmes conditions avec l'amiodarone ont montré que ce composé ne protège que 75% des animaux à 15 mg/kg.

En outre, les Composés 1, 4 et 5 ont montré une protection très hautement significative vis-à-vis de la tachycardie ventriculaire par perfusion d'aconitine chez le rat et ce, dès la dose de 5 mg/kg par voie intraveineuse, la $DA_{50}$ protectrice de chacun de ces composés étant de 2,5 mg/kg.

De plus, les Composés 1 et 5 se sont révélés actifs vis-a-vis de la tachycardie ventriculaire provoquée chez le chien par l'administration intraveineuse de ouabaïne puisqu'ils restaurent le rythme sinusal à la dose cumulée de 10 à 15 mg/kg par voie intraveineuse pendant plus de 90 minutes. Des tests de toxicité ont également été effectués avec les composés selon l'invention lesquels ont montré la toxicité relativement faible de ces composés, les rendant potentiellement utilisables comme médicaments.

Les dérivés de benzofuranne de formule I peuvent être préparés au départ de dérivés de benzoyl benzofuranne de formule générale:

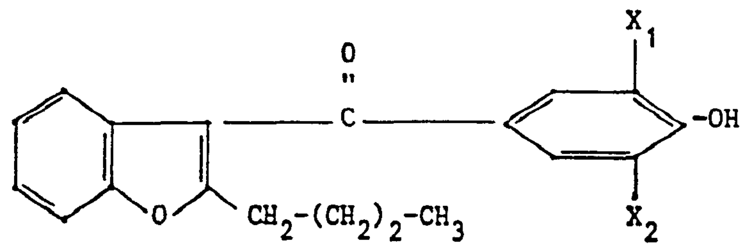

par condensation de ces dérivés avec:

a) le chloro-1 diéthylamino-2 éthane en présence d'un carbonate de métal alcalin, par exemple le carbonate de potassium et dans un milieu approprié tel que le benzène pour obtenir les composés de formule I dans laquelle R représente un radical monoéthylamino ou diéthylamino

b) le dibromo-1,2 éthane en présence d'un carbonate de métal alcalin tel que par exemple le carbonate de potassium dans un milieu approprié par exemple la méthyl éthyl cétone, suivie de l'aminolyse du dérivé bromoéthoxy obtenu, par l'éthylamine en milieu alcoolique par exemple en milieu éthanolique, pour obtenir les composés de formule I dans laquelle R représente un radical monoéthylamino ou diéthylamino.

c) le chloro-1 triphénylméthylamino-2 éthane en milieu basique dans un solvant tel que le N,N-diméthylformamide suivie du clivage du groupement triphénylméthyle par passage en milieu acide, pour obtenir les composés de formule I dans laquelle R représente un groupement amino.

Quant aux sels d'addition non toxiques, ceux-ci peuvent être préparés, selon des méthodes classiques, en faisant réagir les susdits dérivés de benzofuranne de formule I avec l'acide organique ou inorganique approprié, par exemple l'acide oxalique ou chlorhydrique.

Les composés selon l'invention peuvent être administrés, en tant que principe actif médicamentaux sous forme de compositions unitaires convenant à l'administration en thérapie humaine ou vétérinaire. Pour ce qui concerne l'unité d'administration, celle-ci peut prendre la forme, par exemple d'un comprimé, d'une dragée, d'une capsule, d'une gélule, d'une poudre, d'une suspension ou d'un sirop pour l'administration orale, d'un suppositoire pour l'administration rectale, d'une solution stérile ou suspension pour l'administration parentérale.

La quantité de principe actif en question par unité d'administration varie selon que cette unité est destinée à l'administration orale, rectale ou parentérale. Dans le cas d'administration orale, l'unité comprend de 50 à 300 mg, dans le cas d'administration rectale de 50 à 200 mg et dans le cas d'administration parentérale de 50 à 150 mg de principe actif.

L'administration journalière de compositions de l'invention dépend de la voie d'administration choisie.

Généralement, la quantité de principe actif peut varier de 50 à 1200 mg par jour à un être humain adulte.

Sous son aspect le plus général et en tenant compte de la voie d'administration choisie, les compositions thérapeutiques de l'invention sont préparées en associant le principe actif constitué d'au moins un composé selon l'invention à savoir un dérivé de benzofuranne de formule I ou un de ses sels d'addition non toxiques, avec un véhicule pharmaceutique ou un excipient approprié, ce dernier pouvant être sélectionné parmi les substances telles que l'eau distillée, l'alcool benzylique, le lactose, les amidons, le talc, le stéarate de magnésium, la polyvinylpyrrolidone, l'acide alginique, la silice colloïdale, le dioxyde de titane, les agents édulcorants etc... On décrit par la suite, de manière plus particulière mais non limitative, quelques modes de réalisation de compositions de l'invention.

On prépare, par exemple, une composition de l'invention sous forme decomprimé en melangeant le principe actif constitué d'au moins un composé selon l'invention avec un véhicule pharmaceutique ou un excipient approprié par exemple le mannitol, l'amidon de maïs ou la polyvinylpyrrolidone ou analogue, puis en granulant

et en comprimant en présence d'un véhicule pharmaceutique tel que l'amidon de maïs, le stéarate de magnésium ou analogues. Si nécessaire, les comprimés ainsi obtenus peuvent être dragéifiés, enrobés d'un vernis à délitage entérique ou encore enrobés de manière telle que le principe actif se libère graduellement.

De même, on obtient une composition de l'invention sous forme de gélule par simple mélange du principe actif en question avec un véhicule pharmaceutique, ce mélange étant par la suite introduit dans des gélules molles ou dures. On peut également procéder au départ du granulé destiné à la préparation d'une composition sous forme de comprimé.

En outre, on peut préparer une composition pour administration par voie rectale sous forme de suppositoire en coulant, dans un moule approprié, un mélange formé du principe actif en question et d'un agent liant tel que le Witepsol "S 55" (Witepsol est une marque appartenant à Dynamit Nobel A.-G.) ou la lanoline.

Pour une administration parentérale, on réalise des solutions aqueuses stériles et injectables contenant le principe actif en question ainsi qu'un adjuvant de solubilisation, si nécessaire, par exemple le polysorbate 80 et un agent de conservation tel que l'alcool benzylique.

Les composés selon l'invention peuvent être utilisés également dans des compositions pharmaceutiques ou vétérinaires en association avec d'autres ingrédients actifs selon le résultat thérapeutique recherché.

On peut envisager, à titre d'exemple, des compositions contenant outre le principe actif, un agent tranquillisant ou sédatif du système nerveux central tel qu'un dérivé de benzodiazépine comme le diazepam ou un dérivé d'acide barbiturique tel que le pentobarbital.

A titre d'exemples non limitatifs, on décrira, par la suite, la préparation des composés selon l'invention ainsi que de compositions les contenant:

**EXEMPLE 1**

**Oxalate acide de n-butyl-2[iodo-3(diéthylamino-2 éthoxy)-4 benzoyl]-3 benzofuranne**

Dans un ballon tricol, on chauffe au reflux 105 g (0,25 mole) de n-butyl-2 (iodo-3 hydroxy-4 benzoyl)-3 benzofuranne et 103,5 g de carbonate de potassium dans 1,5 l de benzène pendant 30 min.

On ajoute ensuite 10 ml d'eau puis une solution de 43 g de chlorhydrate de chloro-1 diéthylamino-2 éthane dans 17,5 ml d'eau. L'addition terminée, on maintient le reflux pendant 4 h avec élimination d'eau au moyen d'un système Dean-Stark.

On refroidit, on décante la phase benzénique et on la lave avec trois fois 75 ml d'eau jusqu'à neutralité.

On élimine le benzène sous vide et on reprend le résidu avec un peu d'acétate d'éthyle. On forme alors l'oxalate par addition d'une solution d'acide oxalique dans l'isopropanol.

On recristallise l'oxalate obtenu dans un mélange isopropanol/acétate d'éthyle pour isoler environ 75 g d'oxalate acide de n-butyl-2[iodo-3 (diéthylamino-2 éthoxy)-4 benzoyl]-3 benzofuranne.

P.F.: 101 ± 1°C.

**EXEMPLE 2**

**Oxalate acide de n-butyl-2[(éthylamino-2 éthoxy)-4 benzoyl]-3 benzofuranne.**

**a) n-Butyl-2[(bromo-2 éthoxy)-4 benzoyl]-3 benzofuranne**

Dans un ballon tricol de 1 l muni d'un réfrigérant ascendant et d'un agitateur mécanique, on introduit 20,6 g (0,07 mole) de n-butyl-2(hydroxy-4 benzoyl)-3 benzofuranne, 39,2 g de carbonate de potassium anhydre et 200 ml de méthyl éthyl cétone. On agite et chauffe ce mélange à ébullition pendant une heure. On laisse refroidir partiellement et on ajoute 65,8g (0,35 mole) de dibromo-1,2 éthane. On poursuit ensuite le chauffage à ébullition pendant 6 heures.

Après refroidissement, on filtre et lave le solide avec de la méthyl éthyl cétone.

On chasse le solvant sous vide et on obtient ainsi un résidu huileux épais. On le purifie alors par chromatographie sèche sur colonne de silice en utilisant le choroforme comme solvant.

On obtient ainsi 20,4 g de n-butyl-2[(bromo-2 éthoxy)-4 benzoyl]-3 benzofuranne sous forme d'une huile de 98% de pureté.

Rendement: 72,6%

Suivant le même procédé, on a préparé les composés suivants: n-Butyl-2[(bromo-2 éthoxy)-4 iodo-3 benzoyl]-3 benzofuranne (huile)

Rendement: 92%

n-Butyl-2[(bromo-2 éthoxy)-4 diiodo-3,5 benzoyl]-3 benzofuranne

Rendement: 82%

P.F.: 86°C

**b) Oxalate acide de n-butyl-2[(éthylamino-2 éthoxy)-4 benzoyl]-3 benzofuranne.**

On chauffe à 100°C pendant 20h, dans une ampoule scellée, 8 g (0,02 mole) de n-butyl-2[(bromo-2 éthoxy)-4 benzoyl]-3 benzofuranne, 75 ml d'éthanol et 10 ml d'une solution d'éthylamine à environ 33% dans l'éthanol.

On distille l'éthanol et l'excès d'éthylamine au rotavapor et on dissout le résidu huileux dans le dichloréthane.

On lave ensuite la phase dichloréthane avec une solution d'hydroxyde de sodium à 10% puis deux fois à l'eau.

On sèche avec du sulfate de sodium anhydre, filtre et chasse le solvant sous pression réduite. On reprend le résidu huileux à l'éther éthylique sec, filtre et forme l'oxalate par addition d'une solution d'acide oxalique anhydre dans l'éther éthylique.

Après deux recristallisations dans l'éthanol absolu, on obtient 5,1 g d'oxalate acide de n-butyl-2[(éthylamino-2 éthoxy)-4 benzoyl]-3 benzofuranne.

Rendement: 56% (pureté: 99,2%)

P.F.: 204°C - Chlorhydrate: P.F.: 158-159°C.

En suivant le même procédé, on a préparé les composés suivants:

Oxalate de n-butyl-2[(éthylamino-2 éthoxy)-4 iodo-3 benzoyl]-3 benzofuranne

Rendement: 18%

P.F.: 183°C

Oxalate de n-butyl-2[(éthylamino-2 éthoxy)-4 diiodo-3,5 benzoyl]-3 benzofuranne

Rendement: 31%

P.F.: 195°C

Chlorhydrate de n-butyl-2[(éthylamino-2 éthoxy)-4 diiodo-3,5 benzoyl]-3 benzofuranne

P.F.: 175°C

**EXEMPLE 3**

**Oxalate de n-butyl-2[(amino-2 éthoxy)-4 benzoyl]-3 benzofuranne**

On laisse réagir 0,5 g (0,022 at.gr.) de sodium avec 160 ml de méthanol et on distille ensuite le méthanol sous pression réduite. Après sèchage sous haut vide du méthylate de sodium ainsi obtenu, on le dissout dans 100 ml de N,N-diméthylformamide et on ajoute, à cette solution, 6 g (0,02 mole) de n-butyl-2(hydroxy-4 benzoyl)-3 benzofuranne.

Après dissolution de ce composé, on ajoute 7,5 g (0,032 mole) de chlorure de tritylaminoéthyle, on agite et on chauffe à 120°C pendant 5 heures. Après refroidissement, on verse le produit de la réaction dans 1000 ml d'eau. On obtient une émulsion qui, détruite par addition de chlorure de sodium, donne naissance à un précipité gommeux. On le dissout dans 120 ml d'une solution acide acétique/eau 9/1 et on porte cette solution à l'ébullition pendant 1 minute. Au cours du refroidissement, le triphénylcarbinol cristallise. On filtre, lave les cristaux avec de l'acide acétique et on distille, au rotavapor, l'acide acétique/eau du filtrat. On dissout le résidu dans le chloroforme et on lave la solution chloroformique d'abord avec une solution d'hydroxyde de sodium à 10% ensuite deux fois avec de l'eau. On sèche sur sulfate de sodium anhydre, filtre et chasse le chloroforme au rotavapor. On dissout le résidu dans l'éther éthylique sec, on filtre et on forme l'oxalate par addition d'une solution d'acide oxalique anhydre dans l'éther éthylique. On obtient ainsi 3,1 g d'oxalate brut. Après recristallisation dans l'éthanol, on obtient 2,3 g d'oxalate de n-butyl-2[(amino-2 éthoxy)-4 benzoyl]-3 benzofuranne.

Rendement: 26,9%

P.F.: 128°C

En suivant le même procédé, on a préparé les composés suivants:

Oxalate de n-butyl-2[(amino-2 éthoxy)-4 iodo-3 benzoyl]-3 benzofuranne

Rendement: 21,7%

P.F.: 146°C

Oxalate de n-butyl-2[(amino-2 éthoxy)-4 diiodo-3,5 benzoyl]-3 benzofuranne

Rendement: 20,6%

P.F.: 218°C.

## EXEMPLE 4

### Compositions administrables par voie orale

#### I. Comprimé

a) On a préparé des comprimés à croquer ayant la composition ci-dessous:
Composé selon l'invention 100 mg
Mannitol 141,5 mg
Amidon de Maïs 30 mg
Polyvinylpyrrolidone 12mg
Saccharine sodique 0,5 mg
Cyclamate sodique 5 mg
Acide alginique 8 mg
Stéarate de magnésium 3 mg
et ce de la manière suivante:

On mélange 200 g de composé selon l'invention avec 283 g de mannitol, 40 g d'amidon de maïs et 10 g de cyclamate sodique puis on tamise les poudres. On dissout 24 g de polyvinylpyrrolidone et 1 g de saccharine sodique dans 120 ml d'eau distillée, on malaxe les poudres avec la solution de polyvinylpyrrolidone et on granule. On sèche le granulé pendant 8 heures à 40-50°C puis on le calibre. Au granulé calibré, on ajoute 20 g d'amidon de maïs, 16 g d'acide alginique et 6 g de stéarate de magnésium. On comprime alors au poids unitaire de 300 mg.

b) D'une manière semblable, on a également préparé des comprimés ayant la composition suivante:
Composé selon l'invention 200 mg
Lactose 96 mg
Amidon de maïs 60 mg
Polyvinylpyrrolidone 12 mg
Silice colloïdale 2,4 mg
Stéarate de magnésium 4,6 mg
375 mg

c) On a préparé des comprimés dragéifiés au départ de comprimés de composition suivante:
Composé selon l'invention 150 mg
Lactose 100 mg
Amidon de maïs 54 mg
Polyvinylpyrrolidone 10 mg
Stéarate de magnésium 4 mg
Silice colloïdale 2 mg
en les recouvrant de polivinylpyrrolidone, de polyéthylèneglycol 400 et de talc, puis en leur appliquant un vernis formé de méthylcellulose, éthylcellulose, diéthylphtalate, dioxyde de titane et hydroxypropylméthylcellulose.

#### II. Gélule

On a prélevé du granulé obtenu au paragraphe Ib ci-dessus et on l'a introduit dans des gélules molles pour obtenir une composition unitaire identique à celle des comprimés du paragraphe Ib en question.

## EXEMPLE 5

### Composition administrable par voie rectale

On a préparé des suppositoires en coulant, dans des moules en chlorure de polyvinyle, le mélange ayant la composition suivante:
Composé selon l'invention 200 mg
Witepsol "S 55" 1030 mg
Lanoline anhydre 90 mg
1320 mg

**EXEMPLE 6**

**Composition administrable par voie parentérale**

Une composition aqueuse stérile injectable a été préparée, répondant à la formulation suivante:
Composé selon l'invention 150 mg
Polysorbate 80 300 mg
Alcool benzylique 60,6 mg
Eau pour préparations injectables q.s. pour 3 ml laquelle a été introduite dans une ampoule adéquate avec l'azote comme gaz protecteur.

**Revendications**

1. Composition pharmaceutique ou vétérinaire destinée à combattre des troubles ischémiques cardiaques caractérisée en ce qu'elle contient comme principe actif essentiel, au moins un dérivé de benzofuranne de formule géneralé:

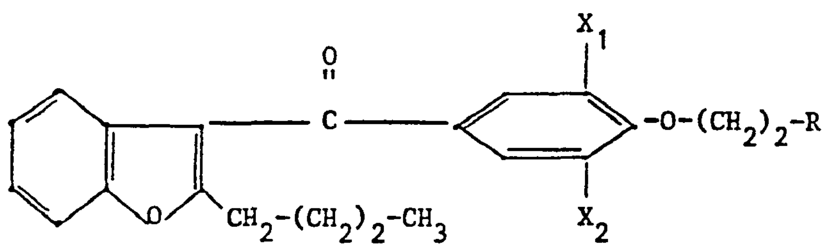

ou un de ses sels d'addition non toxique, dans laquelle R représente un radical amino, monoéthylamino ou diéthylamino et $X_1$ et $X_2$, qui sont identiques ou différents, représentent hydrogène ou iode à condition que lorsque R représente un radical diéthylamino, $X_1$ et $X_2$ sont différents, en association avec un véhicule pharmaceutique ou un excipient approprié.

2. Composition selon la Revendication 1 caractérisée en ce que le sel d'addition non toxique est le chlorhydrate ou l'oxalate acide.

3. Composition selon la Revendication 1 caractérisée en ce qu'elle contient le n-butyl-2[(éthylamino-2 éthoxy)-4 benzoyl]-3 benzofuranne ou un de ses sels d'addition non toxiques.

4. Composition selon la Revendication 1 caractérisée en ce qu'elle contient le n-butyl-2[(éthylamino-2 éthoxy)-4 diiodo-3,5 benzoyl]-3 benzofuranne ou un de ses sels d'addition non toxiques.

5. Composition selon l'une des Revendications 1 à 4 caractérisée en ce qu'elle se présente sous forme d'unité d'administration pour l'administration orale.

6. Composition selon l'une des Revendications 1 à 4 caractérisée en ce qu'elle se présente sous forme d'unité d'administration pour l'administration rectale.

7. Composition selon l'une des Revendications 1 à 4 caractérisée en ce qu'elle se présente sous forme d'unité d'administration pour l'administration parentérale.

8. Composition selon l'une des Revendications 1 à 7 caractérisée en ce qu'elle se présente sous forme d'unité d'administration contenant de 50 à 300 mg de dérivé de benzofuranne ou de ses sels d'addition non toxiques.

**Patentansprüche**

1.- Pharmazeutische oder veterinärmedizinische Zusammensetzung zur Behandlung ischämischer cardialer Störungen, enthaltend als wesentlichen aktiven Bestandteil mindestens ein Benzofuranderivat der allgemeinen Formel

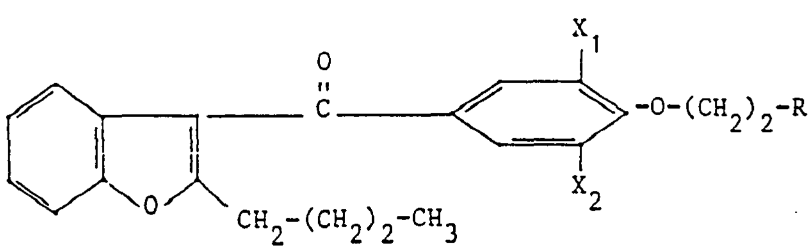

oder ein pharmazeutisch annehmbares Säure-Additionssalz desselben, worin R einen Amino-, Monoethylamino- oder Diethylaminorest bedeutet und $X_1$ und $X_2$, die gleich oder verschieden sind, jeweils Wasserstoff oder Jod bedeuten, unter der Bedingung, daß - wenn R einen Diethylaminorest darstellt - $X_1$ und $X_2$ verschieden sind, in Verbindung mit einem geeigneten pharmazeutischen Träger oder Hilfsstoff.

2.- Zusammensetzung nach Anspruch 1, worin das pharmazeutisch annehmbare Säure-Additionssalz das Hydrochlorid oder Hydrogenoxalat ist.

3.- Zusammensetzung nach Anspruch 1, worin der wesentliche aktive Bestandteil 2-n-Butyl-3-[4-(2-ethylaminoethoxy)-benzoyl]-benzofuran oder ein pharmazeutisch annehmbares Säure-Additionssalz desselben ist.

4.- Zusammensetzung nach Anspruch 1, worin der wesentliche aktive Bestandteil 2-n-Butyl-3-[4-(2-ethylaminoethoxy)-3,5-dijod-benzoyl]-benzofuran oder ein pharmazeutisch annehmbares Säure-Additionssalz desselben ist.

5.- Zusammensetzungen nach einem der Ansprüche 1 bis 4, wobei die Zusammensetzung in Form einer Dosierungseinheit vorliegt, die zur oralen Verabreichung geeignet ist.

6.- Zusammensetzung nach einem der Ansprüche 1 bis 4, die in Form einer Dosierungseinheit, welche zur rektalen Verabreichung geeignet ist, vorliegt.

7.- Zusammensetzung nach einem der Ansprüche 1 bis 4, die in Form einer Dosierungseinheit, welche zur parenteralen Verabreichung geeignet ist, vorliegt.

8.- Zusammensetzung nach einem der Ansprüche 1 bis 7, die in Form einer Dosierungseinheit vorliegt, die 50 bis 300 mg aktiven Bestandteil enthält.

## Claims

1. Pharmaceutical or veterinary composition for the treatment of ischemic cardiac disorders, the said composition containing as essential active ingredient at least one benzofuran derivative of the general formula:

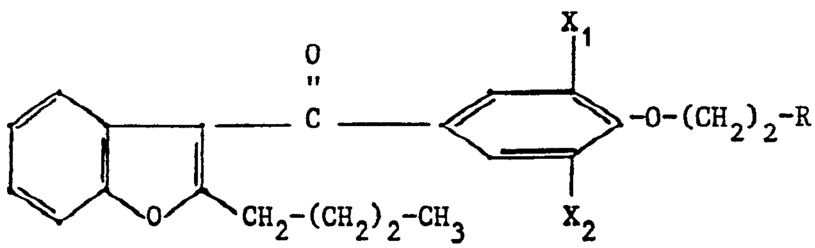

or a pharmaceutically acceptable acid addition salt thereof, wherein R represents an amino, monoethylamino or diethylamino radical and $X_1$ and $X_2$, which are the same or different, each represent hydrogen or iodine, with the proviso that when R represents a diethylamino radical, $X_1$ and $X_2$ are different, in association with an appropriate pharmaceutical carrier or excipient.

2. Composition according to Claim 1 wherein the pharmaceutically acceptable acid addition salt is the hydrochloride or acid oxalate.

3. Composition according to Claim 1 wherein the essential active ingredient is 2-n-butyl-3-[4-(2-ethylaminoethoxy)-benzoyl]-benzofuran or a pharmaceutically acceptable acid addition salt thereof.

4. Composition according to Claim 1 wherein the essential active ingredient is 2-n-butyl-3-[4-(2-ethylaminoethoxy)-3,5-diiodo-benzoyl]-benzofuran or a pharmaceutically acceptable acid addition salt thereof.

5. Composition according to any one of Claims 1 to 4, the said composition being in a dosage unit form suitable for oral administration.

6. Composition according to any one of Claims 1 to 4, the said composition being in a dosage unit form suitable for rectal administration.

7. Composition according to any one of Claims 1 to 4, the said composition being in a dosage unit form suitable for parenteral administration.

8. Composition according to any one of Claims 1 to 7, said composition being in a dosage unit form containing from 50 to 300 mg of active ingredient.